# EUROPEAN PATENT SPECIFICATION

(11) **EP 0 395 102 B1**
(45) Date of publication and mention of the grant of the patent: **31.03.1993**
(21) Application number: 90108108.3
(22) Date of filing: 27.04.1990
(51) Int. Cl.: C07C 309/81, C07C 303/22

(54) **Process for preparing perfluoroalkenyl sulfonyl fluorides**
Verfahren zur Herstellung von Perfluoroalkenyl-sulfonyl-Fluoriden
Procédé pour la préparation de fluorures de perfluoroalkenyl sulfonyle

(30) Priority: 28.04.1989 IT 2033989
(43) Date of publication of application: 31.10.1990
(73) Proprietor: AUSIMONT S.p.A., I-20121 Milano (IT)
(72) Inventor: Navarrini, Walter, Dr., I-20010 Boffalora Ticino, Milan (IT); Modena, Silvana, Dr., I-20052 Monza, Milan (IT)
(74) Representative: Barz, Peter, Dr.

(56) References cited:
- GB-A- 2 027 709
- US-A- 3 041 317
- US-A- 3 714 245
- US-A- 3 718 627
- JOURNAL OF THE CHEMICAL SOCIETY. 1966, LETCHWORTH GB pages 1171 - 1179; R. E. BANKS ET AL.: "PERFLUOROALKYL DERIVATIVES OF SULPHUR. PART VIII. SYNTHESIS AND REACTIONS OF PERFLUOROETHYLENESULPHONYL FLUORIDE"
- JOURNAL OF THE AMERICAN CHEMICAL SOCIETY. vol. 82, no. 23, 14 December 1960, GASTON, PA US pages 6181 - 6188; D.C. ENGLAND ET AL.: "REACTIONS OF FLUOROÖLEFINS WITH SULFUR TRIOXIDE."

## Description

The present invention relates to a process for the preparation of perfluoroalkenyl sulfonyl fluorides, in particular of perfluorovinyl sulfonyl fluoride.

These perfluorinated compounds which contain the sulfonyl function are useful monomers for the preparation of high-molecular-weight polymers which find several uses.

US-A-3,041,317 discloses a process for the synthesis of perfluoroalkenyl sulfonyl fluorides of formula

R_{f}-CF=CF-SO₂F

wherein R_{f} is F, a perfluoroalkyl radical or an omega-hydro-perfluoroalkyl radical. The starting materials for said synthesis are 2-hydro-perfluoroalkyl-sulfonyl fluorides of general formula

R_{f}-CF₂-CFH-SO₂F

which are dehydrofluorinated in order to yield perfluoroalkenyl sulfonyl fluorides. The reaction is carried out in a plant operating under reduced pressure, inside which a stream of reactants flows towards a catalyst composed of chromium oxide supported on KCl, at temperatures of from 450 to 630°C.

In particular for vinyl-sulfonyl fluoride, the reaction is carried out at temperatures of from 508 to 517°C, resulting in a conversion of 51% and a yield of 61%, based on the converted reactants, per each individual run.

R.E. Banks in J. Chem. Soc. (C), 1966 reports a synthesis of perfluorovinyl sulfonyl fluoride wherein a catalyst and operating conditions are used which are exactly the same as those disclosed in US-A-3,041,317, with the only difference that the catalytic bed is pre-heated at 510°C before the synthesis is carried out.

In the paper by R.E. Banks, the general reactivity of said molecule is furthermore described.

In both of the above processes the starting products are 2-hydro-perfluoro-sulfonyl fluorides having the general formula

R_{f}-CHF-CF₂-SO₂F

which may be synthesized by means of the controlled hydrolysis of the corresponding sultones of formula 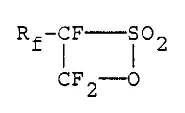
as described by D.C. England in J. Am. Chem. Soc. 1960, 82, 6181.

The main object of the present invention is the provision of a process for preparing perfluoroalkenyl sulfonyl fluorides which can be carried out under temperature and pressure conditions which are more advantageous from an economic point of view than those of the processes known from the prior art.

Another object of the present invention is the provision of a simplified process which requires a smaller number of steps than the processes of the prior art.

A further object is the provision of a process which uses readily available and inexpensive catalysts.

Finally, still a further object of the present invention is the provision of a process which affords high yields of desired perfluoroalkenyl sulfonyl fluorides.

According to the present invention these and still other objects which will become clear from the following disclosure may be achieved by a process for the preparation of perfluoroalkenyl sulfonyl fluorides of general formula (I)

R_{f}-CF=CF-SO₂F (I)

wherein:
R_{f} is F or a perfluoroalkyl radical of from 1 to 9 carbon atoms,
which process comprises the contacting, at a temperature of from about 150 to about 450°C, of a starting material comprising the β-sultone of a 2-hydroxy-1-perfluoroalkyl-1,2,2-trifluoroethane-sulfonic acid of general formula (II): 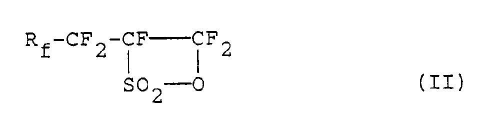
with a reactant selected from the oxides and carbonates of elements of the Groups IA, IIA and IIB, the oxides of elements of the Groups IIIA and IVA of the Periodic Table of Elements and mixtures thereof and isolating the desired compound of formula (I) from the reaction effluent.

It has surprisingly been found that when the reactants and the operating conditions according to the instant invention are used, a perfluoroalkenyl-sulfonyl fluoride (I) can directly be synthesized by starting from a β-sultone (II), without having to pass through further intermediate steps such as, e.g., the hydrolysis required by the processes of the prior art.

The reactant used in the instant process is an oxide and/or carbonate of the above mentioned elements, among which CaO, SiO₂, MgO, ZnO, Na₂CO₃, CaCO₃ and mixtures thereof are generally preferred.

The reactions on which the process of the present invention is based can be illustrated as follows for the case when CaO or CaCO₃ are used:

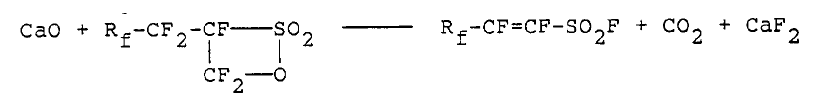

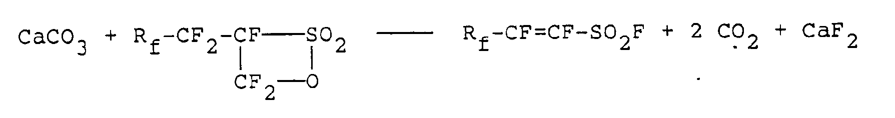

The amount of reactant used generally is at least the stoichiometric amount required for the above reactions. Preferably the reactant is used in excess over said stoichiometric amount, also depending on the degree of comminution of the reactant.

The reactant advantageously is subjected to a preliminary activation by contacting it with the starting material before the actual process is carried out.

The process according to the present invention can advantageously be carried out continuously under atmospheric pressure, although pressures lower or higher than atmospheric pressure can be used as well.

The process temperature ranges from about 150 to about 450°C, preferably from about 180 to about 280°C.

The process is suitably carried out by causing a stream of an inert gas carrier saturated with vapours of the sultone used as the starting material to pass or flow over or through a bed of the reactant contained inside a glass or steel column reactor.

As the inert carrier, nitrogen is preferably used.

The process usually is carried out under anhydrous conditions, with both nitrogen and the reactant bed being previously dried thoroughly. The reactant bed may be thoroughly dried by heating it at about 300-330°C for a few hours.

In the reactor, a packing of a particulate, e.g., siliceous, material with small particle size, is preferably used in addition to the reactant bed.

Flow rates of the carrier and starting material stream such as to secure a residence time of the starting material in the reactor of from about 3 to about 30 seconds are generally used.

The effluent from the reactor may be condensed at a temperature of from 0 to -196°C in order to separate the reaction products from nitrogen, which can be discharged.

The condensate may be distilled under reduced pressure, and the vapours may be condensed in a first condenser at a higher temperature, e.g., at a temperature of from -100 to -20°C in order to recover the desired perfluoroalkenyl sulfonyl fluoride, and then in a second condenser at -110°C in order to revcover the SO₂ produced. The other byproducts, such as CO₂ and SiF₄ as well as any decomposition products, such as CF₃COF or C₂F₄, may be removed by applying a dynamic vacuum during said distillation.

A complete conversion of the starting material may be obtained, with yields, defined as the ratio of the mols of desired product to the reacted mols of starting material (sultone), as high as 65 to 75%.

The process according to the present invention is particularly useful for the preparation of compounds of formula (I) wherein R_{f} is F or a perfluoroalkyl radical with 1 to 3 carbon atoms, in particular CF₃ and C₂F₅.

The following examples are to illustrate preferred embodiments of the present invention.

### EXAMPLE 1

In a continuously working plant 3.2 g (13.5 mmol) of the sultone of 2-hydroxy-1-trifluoromethyl-1,2,2-trifluoroethane sulfonic acid of formula 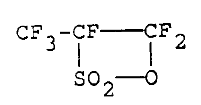
were introduced into a loading "U"-shaped trap.

Said loading trap was then connected to the continuous plant by means of valves.

A stream of previously thoroughly dried nitrogen was caused to flow (flow rate 18 litres/hour) through the loading trap kept at -35°C throughout the entire experiment.

Nitrogen saturated with the vapours of the sultone used as the starting material was passed through the reactor which was kept at 210°C.

Within about 3 hours, all of the starting material was passed through the reactor (a steel column having a diameter of 3.0 cm, packed with 100 g of calcium oxide and 100 g of small steel tubes). The reactant bed thus obtained preliminarily was thoroughly dried by keeping it at 320°C for 4 hours, and was then activated by causing 13.5 mmol of β-sultone of perfluoropropene to flow through it under the same operating conditions as described above.

The effluent gases from the reactor were passed through two traps maintained at -196°C in order to condense all of the reaction products, whereas the nitrogen carrier gas was discharged.

The crude reaction product contained in the two traps was distilled under a pressure of 10⁻³ torr. The vapours coming from the still kettle were caused to flow through cold traps kept at -80 and -110°C, respectively.

Inside the trap at -110°C, 0.2 mmol of SO₂ condensed. In the trap at 80°C 9.4 mmol of the desired trifluorovinyl sulfonyl fluoride of formula

CF₂=CF-SO₂F

were obtained. CO₂ and any decomposition products, such as, e.g., CF₃COF and C₂F₄ were removed by applying a dynamic vacuum during the distillation.

The conversion of the starting material was complete.

The yield, defined as the ratio of mols of desired product (CF₂=CF-SO₂F) to the reacted mols of starting material, was 70%.

### EXAMPLE 2

By following the procedure of Example 1, 3.15 g (13.5 mmol) of sultone of 2-hydroxy-1-trifluoromethyl-1,2,2-trifluoroethane-sulfonic acid were caused to flow through a reactant bed of CaCO₃, activated as in Example 1 and maintained at 250°C throughout the entire experiment.

The flow rate of the carrier nitrogen stream was 10 litres/hour throughout the entire experiment.

The reaction yield, defined as in Exmaple 1, was 15%.

### EXAMPLE 3

By following the procedure described in Example 1, but using the sultone of 2-hydroxy-1-pentafluoroethyl-1,2,2-trifluoroethane-sulfonic acid as the starting material, perfluoropropenyl-sulfonyl fluoride was prepared.

## Claims

1. Process for preparing perfluoroalkenyl sulfonyl fluorides of general formula (I)
R_{f}-CF=CF-SO₂F (I)
wherein:
R_{f} is selected from F and perfluoroalkyl radicals of from 1 to 9 carbon atoms;
comprising contacting, at a temperature of from about 150 to about 450°C, a starting material comprising the β-sultone of a 2-hydroxy-1-perfluoroalkyl-1,2,2-trifluoroethane-sulfonic acid of formula (II); 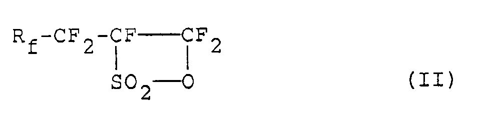 wherein R_{f} is as defined above; with a reactant selected from the oxides and carbonates of elements of the Groups IA, IIA and IIB and the oxides of elements of the Groups IIIA and IVA of the Periodic Table of Elements, and mixtures thereof.

2. Process according to claim 1, wherein R_{f} is F or a perfluoroalkyl radical of from 1 to 3 carbon atoms.

3. Process according to any one of claims 1 and 2, carried out continuously under atmospheric pressure.

4. Process according to any one of claims 1 to 3, wherein said reactant is selected from CaO, MgO, ZnO, SiO₂, Na₂CO₃, CaCO₃ and mixtures thereof.

5. Process according to any one of claims 1 to 4, wherein the temperature ranges from about 180 to about 280°C.

6. Process according to any one of claims 1 to 5, wherein the contacting with the reactant is carried out by causing a stream of an inert gas carrier saturated with the vapours of said starting sultone of formula (II) to flow over or through the reactant.

7. Process according to claim 6, wherein said inert gas carrier is nitrogen.

## Patentansprüche

1. Verfahren zur Herstellung von Perfluoralkenylsulfonylfluoriden der allgemeinen Formel (I)
R_{f}-CF=CF-SO₂F (I)
worin:
R_{f} ausgewählt ist aus F und Perfluoralkylresten mit 1 bis 9 Kohlenstoffatomen;
umfassend das In-Berührung-Bringen, bei einer Temperatur von etwa 150 bis etwa 450°C, eines Ausgangsmaterials, das das β-Sulton einer 2-hydroxy-1-perfluoralkyl-1,2,2-trifluorethansulfonsäure der Formel (II) umfaßt: 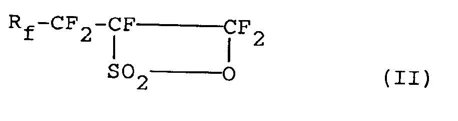 worin:
R_{f} wie oben definiert ist; mit einem Reaktanten, der ausgewählt ist aus den Oxiden und Carbonaten von Elementen der Gruppen IA, IIA und IIB und den Oxiden von Elementen der Gruppen IIIA und IVA des Periodensystems der Elemente und Mischungen davon.

2. Verfahren nach Anspruch 1, bei welchem R_{f} für F oder einen Perfluoralkylrest mit 1 bis 3 Kohlenstoffatomen steht.

3. Verfahren nach irgendeinem der Ansprüche 1 und 2, das kontinuierlich unter atmosphärischem Druck durchgeführt wird.

4. Verfahren nach irgendeinem der Ansprüche 1 bis 3, bei welchem der Reaktant ausgewählt ist aus CaO, MgO, ZnO, SiO₂, Na₂CO₃, CaCO₃ und Mischungen davon.

5. Verfahren nach irgendeinem der Ansprüche 1 bis 4, bei welchem die Temperatur im Bereich von etwa 180 bis etwa 280°C liegt.

6. Verfahren nach irgendeinem der Ansprüche 1 bis 5, bei welchem das In-Berührung-Bringen mit dem Reaktanten durchgeführt wird, indem man einen Strom eines Inertgasträgers, der mit den Dämpfen des Ausgangssultons der Formel (II) gesättigt ist, veranlaßt, über oder durch den Reaktanten zu fließen.

7. Verfahren nach Anspruch 6, bei welchem der Inertgasträger Stickstoff ist.

## Revendications

1. Procédé pour la préparation de fluorures de perfluoroalcénylsulfonyle de formule générale (I):
R_{f}-CF=CF-SO₂F (I)
dans laquelle:
R_{f} est un atome de fluor ou un radical perfluoroalkyle ayant de 1 à 9 atomes de carbone,
comprenant la mise en contact, à une température d'environ 150 à environ 450°C, d'un matériau de départ comprenant la β-sultone d'un acide 2-hydroxy-1-perfluoroalkyl-1,2,2,-trifluoroéthane sulfonique de formule (II): 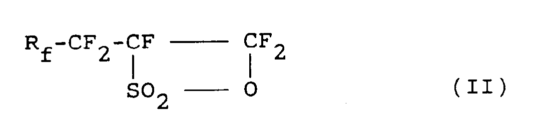 dans laquelle:
R_{f} est tel que défini plus haut;
avec un réactif choisi parmi les oxydes et les carbonates d'éléments des groupes IA, IIA et IIB et les oxydes d'éléments des groupes IIIA et IVA du Tableau Périodique des Eléments et leurs mélanges.

2. Procédé suivant la revendication 1, dans lequel R_{f} est un atome de fluor ou un radical perfluoroalkyle ayant de 1 à 3 atomes de carbone.

3. Procédé suivant l'une quelconque des revendications 1 et 2, effectué en continu à la pression atmosphérique.

4. Procédé suivant l'une quelconque des revendications 1 à 3, dans lequel ce réactif est choisi parmi CaO, MgO, ZnO, SiO₂, Na₂CO₃, CaCO₃ et leurs mélanges.

5. Procédé suivant l'une quelconque des revendications 1 à 4, dans lequel la température est comprise entre environ 180 et environ 280°C.

6. Procédé suivant l'une quelconque des revendications 1 à 5, dans lequel la mise en contact avec le réactif est effectuée par passage d'un courant d'un gaz porteur inerte saturé avec les vapeurs de sultone de départ de formule (II) sur ou à travers le réactif.

7. Procédé suivant la revendication 6, dans lequel ce gaz porteur inerte est de l'azote.
